# FASCICULE DE BREVET EUROPEEN

(11) **EP 3 897 581 B1**
(45) Date de publication et mention de la délivrance du brevet: **03.04.2024**
(21) Numéro de dépôt: 19828741.9
(22) Date de dépôt: 19.12.2019
(51) Int. Cl.: A61K 9/107, A61K 9/00

(54) **COMPOSITION ÉMOLLIENTE SOUS FORME D'ÉMULSION**
EMOLLIENSZUSAMMENSETZUNG IN EMULSIONSFORM
EMOLLIENT COMPOSITION IN EMULSION FORM

(30) Priorité: 21.12.2018 FR 1873902
(43) Date de publication de la demande: 27.10.2021
(73) Titulaire: PIERRE FABRE MEDICAMENT, 81500 Lavaur (FR)
(72) Inventeur: MUGUET, Valérie, 31400 TOULOUSE (FR); CORDOLIANI, Jean-François, 31570 SAINTE FOY D'AIGREFEUILLE (FR)
(74) Mandataire: Regimbeau
(86) Numéro de dépôt international: PCT/EP2019/086365
(87) Numéro de publication internationale: WO 2020/127773

(56) Documents cités:
- WO-A1-2016/008999
- FR-A1- 2 957 260
- FR-A1- 3 013 985

## Description

La présente invention a pour objet une nouvelle composition sous forme d'émulsion comprenant du glycérol, de la vaseline, et de la paraffine liquide ainsi qu'un système des conservateurs et gélifiants particulier.

Le dessèchement de la peau est un problème courant pour une grande partie de la population. Les origines possibles de cette condition sont multiples et les manifestations particulières variables, avec des symptômes comme des squames (pellicules), des gerçures, des rougeurs ou des démangeaisons. L'état de la sécheresse de la peau peut être transitoire et lié à des conditions comportementales ou environnementales particulières (par exemple utilisation des produits de nettoyage, traitement par certains médicaments, conditions climatiques etc.), mais aussi plus chronique et associé à des perturbations du bon fonctionnement de la peau, notamment dans certaines conditions pathologiques tel que la xérose ou aussi la dermatite atopique, le prurit, l'ichtyose, le psoriasis, ou autres indications.

Une structure clef pour l'hydratation de la peau est formée par la couche cornée (stratum corneum), la couche la plus superficielle de l'épiderme. La couche cornée est composée des cornéocytes, des cellules apoptotiques qui sont le résultat de l'ultime phase de mutation des kératinocytes qui remontent progressivement depuis la couche basale, et de lipides épidermiques, sa partie inférieure constitue une véritable barrière de protection face aux facteurs exogènes (pollution, soleil, froid).

Une des fonctions primordiales de la couche cornée est notamment d'assurer le contenu en humidité de la peau et de la protéger contre la perte d'eau endogène. Des mécanismes divers peuvent être cités dans ce contexte. La couche de cornéocytes avec leur enveloppe protéique cornifiée et la concentration importante des lipides dans l'espace intercellulaire représentent une barrière peu perméable contre la perte de l'humidité de la peau ; un rôle fondamental est attribué notamment aux lipides de la famille des céramides.

Une grande partie de l'eau qui se trouve liée dans la couche cornée est associée aux fibres de kératine des cornéocytes et aux parties hydrophiles des lipides intercellulaires. Les cornéocytes contiennent également un mélange désigné comme « Facteur naturel d'hydratation » (Natural Moisturizing Factor, NMF), comprenant des acides aminés, de l'acide pyrrolidone carboxylique, de l'acide lactique, de l'urée, des ions minéraux, et d'autres substances. Ces molécules, basées sur leur propriétés hygroscopiques et solubles, agissent comme humectants endogènes et contribuent donc à la fixation de l'eau dans la couche cornée et assurent une bonne élasticité de la peau.

Inversement, un défaut dans la capacité du stratum corneum de retenir l'humidité cutanée amène des effets du dessèchement de la peau. Les perturbations dans l'homéostasie de l'hydratation se manifestent à plusieurs niveaux. Comme exemple, des changements dans le contenu de céramides dans la peau et dans leur structure seraient liés à la xérose cutanée ; autres facteurs dans divers états pathophysiologiques concernent la structure de la couche lipidique, la cohésion des cornéocytes, la production du facteur naturel d'hydratation (NMF) ou aussi le fonctionnement des transporteurs cellulaires de l'eau et du glycérol, des aquaporines.

Un pilier du traitement et/ou de la prévention du dessèchement de la peau est l'utilisation des produits hydratants et émollients par voie topique, avec le but de rétablir et maintenir l'hydratation de la peau et assurer sa bonne apparence et son élasticité (souplesse). L'utilisateur trouve à sa disposition un certain nombre de ces préparations, qui peuvent être utile à plusieurs niveaux, notamment dans le contexte de la réhydratation de la peau, de l'empêchement de la perte d'humidité et dans la restauration et la réparation de la barrière cutanée ; les préparations particulières présentent souvent une combinaison de ces fonctions. Par conséquent, ces produits sont composés des différentes classes d'ingrédients tel que les occlusifs, humectants, émollients et régénérateurs des protéines.

Ces préparations pour application topique peuvent être mises à disposition sous forme d'émulsion, avec des ingrédients adaptés qui assurent des bonnes propriétés des formulations par exemple sur le niveau de la stabilité, de la conservation, de la consistance, de l'étalement, de l'homogénéité, de la qualité organoleptique, de l'odeur, du toucher, de l'aspect visuel, et/ou d'autres paramètres. Il existe cependant toujours un besoin d'améliorer ces compositions afin de mieux s'adapter aux attentes des consommateurs, aux nouvelles considérations du risque environnemental ou sanitaire pour certains ingrédients, et aux actualisations des restrictions règlementaires.

En effet, plusieurs substances couramment utilisées dans les préparations dermatologiques et cosmétiques pour application topique ont été remises en question récemment. Il s'agit notamment des agents qui sont utilisés pour améliorer la conservation des compositions à usage cutané, par exemple par la protection contre une contamination microbiologique. Un groupe des produits qui est particulièrement controversé dans le domaine dermatologique et cosmétique est notamment les conservateurs de la classe des parabènes. En effet, les parabènes sont suspectés d'être cancérogènes et de perturber le système hormonal. Les formulateurs doivent donc trouver des alternatives à l'emploi de ces molécules. Des considérations similaires s'appliquent à certains autres conservateurs, souvent associés à des propriétés allergisantes et/ou irritantes pour la peau. On peut citer des produits comme l'hydroxyanisole butylé ou BHA, le phénoxyéthanol, le chlorocrésol, l'acide benzoïque et ses sels (benzoates), le benzyl alcool, et les donneurs de formaldéhyde.

Le brevet FR 2 957 260 A1 divulgue une composition galénique pour application cutanée comprenant de l'eau, de la vaseline, de la paraffine et un agent gélifiant,

Les émulsions comprenant une association de glycérol, vaseline, et paraffine liquide telles que décrites dans les demandes WO2009/138517 et WO2009/138515 sont des compositions particulièrement utiles dans la prise en charge de la sécheresse cutanée. Cependant, ces associations de glycérol, vaseline, et paraffine liquide sont combinées avec le parahydroxybenzoate de propyle ou le chlorocrésol comme conservateurs. Il existe donc un réel besoin en des formules innovantes qui évitent ces ingrédients considérés comme problématiques.

Or, il s'avère difficile de remplacer lesdites substances concernées d'une manière fonctionnellement équivalente tout en gardant le corps de de la formulation avec la combinaison approuvée associant glycérol, vaseline, et paraffine liquide et en maintenant les propriétés organoleptiques et physicochimiques établies. Le choix des conservateurs adaptés est limité, et la modification des composants entraine des problèmes galéniques qui sont difficile à surmonter. Ceci est particulièrement vrai lorsqu'il faut maintenir constantes les concentrations des ingrédients principaux de la formule, comme par exemple pour la formule d'une association de glycérol, vaseline, et paraffine liquide à des concentrations respectives d'environ 15%, environ 8% et environ 2% en poids par rapport au poids total de la formulation, et ceci de surcroit dans les conditions établies, notamment dans le cas d'un pH légèrement supérieur à pH7.

En termes de conservation, la demande WO2016/008999 décrit la conservation par haute pression d'une émulsion pour application cutanée qui contient entre autres du glycérol, de la vaseline blanche et du paraffine liquide, et ceci sans ajout des parabènes ou d'autres produits conservateurs. La méthode de conservation est efficace au niveau microbiologique et d'un point de vue physicochimique. Cependant, l'application de cette technique nécessite l'installation d'un appareillage assez coûteux et n'est pas facilement réalisable. De plus, la conservation et les propriétés rhéologiques de l'émulsion n'est pas garantie pour une utilisation répétée et/ou étalée dans le temps, c.a.d. après ouverture du flacon stérile.

Il existe donc un réel besoin de compositions émollientes et protectrices non stériles pour utilisation cutanée pour tous types de peaux, y compris les peaux sensibles, comprenant l'association de glycérol, vaseline, et paraffine liquide, exemptes de conservateurs irritants ou allergisants et en particulier des parabènes. Ladite composition émolliente et protectrice doit en outre présenter une conservation microbiologique appropriée, comme définie par exemple par les critères A et/ou B de la Pharmacopée Européenne, 8ème édition (2016), chapitre 5.1.3. Enfin cette composition doit présenter des propriétés organoleptiques et de consistance acceptables et stables dans le temps. Il est également attendu qu'elle soit adaptée à une utilisation répétée et que la formulation soit possible à produire au niveau industriel, technique et économique.

De façon surprenante, les inventeurs de la présente demande ont pu mettre au point des compositions particulières et remplissant tous ces critères et plus particulièrement une stabilité microbiologique, physicochimique et rhéologique à long-terme. L'invention est d'autant plus surprenante qu'il a été montré que l'association de conservateurs autres que des parabènes en combinaison avec un système d'agents gélifiants adapté a permis d'atteindre le résultat souhaité.

La présente invention porte donc sur une composition sous forme d'une émulsion huile dans eau ou eau dans huile, comprenant
- de l'eau à une concentration entre 30 et 80% en poids par rapport au poids total de la composition
- du glycérol à une concentration entre 10 et 20%, préférentiellement entre 13 et 17%, en poids par rapport au poids total de la composition,
- de la vaseline à une concentration 3 et 20%, préférentiellement entre 5 et 10%, en poids par rapport au poids total de la composition
- de la paraffine liquide à une concentration entre 0,5 et 5%, encore préférentiellement entre 1 et 3%, en poids par rapport au poids total de la composition,
- au moins un conservateur différent des parabènes choisi dans le groupe consistant en l'hexanediol, l'éthylhexylglycérine, le pentylèneglycol, le butylèneglycol, le 1,2 octanediol (caprylyl glycol) et leurs mélanges,
- au moins 2 agents gélifiants dont un agent gélifiant de type polyacrylamide et un second agent gélifiant choisi dans le groupe consistant en : gomme de xanthane, carbomère 980, carbomère 974, hydroxyéthylcelllulose, hydroxypropylméthylcellulose et leurs mélanges.

Des aspects préférés mais non limitatifs de la composition selon l'invention sont les suivants :
La composition selon l'invention peut être préparée sous la forme d'une émulsion simple eau dans huile (E/H) ou huile dans eau (H/E), d'une émulsion multiple comme par exemple, une émulsion eau dans huile dans eau (E/H/E) ou une émulsion huile dans eau dans huile (H/E/H), ou encore sous la forme d'une hydrodispersion ou une lipodispersion, un gel ou un aérosol. Préférentiellement, la présente invention est préparée sous forme d'une émulsion huile dans eau (H/E).

Les émulsions sont des mélanges intimes de deux substances liquides non miscibles. Ce sont toujours deux liquides qui en situation normale sont non miscibles mais qui vont, par des opérations spécifiques (agitation, mélanges, ajout d'émulsionnants), réussir à avoir un aspect macroscopiquement homogène mais microscopiquement hétérogène. Les émulsions se composent d'une phase grasse, une phase aqueuse et un système émulsifiant approprié.

Dans la phase aqueuse de l'émulsion, la composition selon la présente invention contient de l'eau. Avantageusement, l'eau est comprise entre 30 et 80% en poids par rapport au poids total de la composition. Avantageusement, l'eau utilisée pour la phase aqueuse de l'émulsion peut être une eau distillée ou une eau thermale ayant des propriétés dermato-cosmétiques.

La composition selon l'invention contient du glycérol (ou 1,2,3-propanetriol). Avantageusement, le glycérol présente les critères décrits et contrôlés selon la pharmacopée européenne 8ème édition (2016), monographe n°0496. La concentration en glycérol dans la composition est comprise entre 10 et 20%, préférentiellement entre 13 et 17%, et de manière particulièrement préférée est d'environ 15% en poids par rapport au poids total de la composition.

La composition selon l'invention contient de la vaseline (ou pétrolatum). Avantageusement, la vaseline présente les critères décrits et contrôlés selon la pharmacopée européenne 8ème édition (2016), monographe n°1799. La concentration en vaseline est comprise entre 3 et 20%, préférentiellement entre 5 et 10% et de manière particulièrement préférée est d'environ 8% en poids par rapport au poids total de la composition.

La composition selon l'invention contient de la paraffine liquide (*paraffinum perliquidum*). Avantageusement, la paraffine liquide présente les critères décrits et contrôlés selon la pharmacopée européenne 8ème édition (2016), monographe n°0239. La concentration en paraffine liquide est comprise entre 0,5 et 5%, préférentiellement entre 1 et 3% et de manière particulièrement préférée est d'environ 2% en poids par rapport au poids total de la composition.

Dans la composition selon l'invention, l'association de glycérol, vaseline, paraffine liquide est présente selon une proportion comprise entre 10 et 50%, et préférentiellement entre 20 et 30% en poids par rapport au poids total de la composition. La concentration en glycérol est comprise entre 10 et 20%, préférentiellement entre 13 et 17%, et de manière particulièrement préférée est d'environ 15% en poids par rapport au poids total de la composition. La concentration en vaseline est comprise entre 3 et 20%, préférentiellement entre 5 et 10% et de manière particulièrement préférée est d'environ 8% en poids par rapport au poids total de la composition. La concentration en paraffine liquide est comprise entre 0,5 et 5%, préférentiellement entre 1 et 3% et de manière particulièrement préférée est d'environ 2% en poids par rapport au poids total de la composition.

Avantageusement, la composition selon l'invention comprend environ 15% de glycérol, environ 8% de vaseline, environ 2% de paraffine liquide en poids par rapport au poids total de la composition.

La composition selon l'invention contient un ou plusieurs agents conservateurs, tous différents des parabènes. Le terme « conservateur » ou « agent conservateur » comprends tout agent qui est utilisé couramment pour éviter la pousse des microorganismes et/ou pour éviter une dégradation de la composition en question. De préférence, la composition selon l'invention présente entre 0,01% et 20% de conservateurs en poids par rapport au poids total de la préparation, encore préférentiellement entre 0,1 et 15%.

La présente invention est caractérisée en ce qu'elle contient au moins un conservateur mais ne contient pas de parabènes.

Un objet de l'invention concerne une composition à usage topique qui ne contient pas de parabènes. Par l'expression « ne contient pas » de parabènes, il est entendu au sens de la présente invention, que la composition est essentiellement dénuée de parabènes. La concentration en parabènes pourra ainsi être de l'ordre de traces, c'est-à-dire une concentration de l'ordre de 0%, particulièrement à inférieure à 0,01% en poids par rapport au poids total de la préparation, encore préférentiellement inférieure à 0,003% ou encore inférieure à 0,001% en poids par rapport au poids total de la préparation. De tels pourcentages reflètent le caractère d'absence, ou de traces, de parabènes dans la formulation, sachant en effet que l'on ne peut pas assurer une absence absolue et totale du simple fait de possibles, et minimes, contaminations inévitables en cours de fabrication.

Dans le sens de la présente invention, le terme « parabène », « parabènes » ou « conservateur de type parabène » réfère à un ester de l'acide parahydroxybenzoïque et à ses sels. Notamment, le terme « parabènes » réfère aux esters de l'acide parahydroxybenzoïque comme utilisés couramment comme conservateurs. Le terme « parabènes » correspond donc aux esters de l'acide parahydroxybenzoïque, plus particulièrement les parahydroxybenzoates d'alkyle en C1-C8, c'est-à-dire un ester résultant de la condensation de l'acide parahydroxybenzoïque avec un alcool en C1-C8 ainsi que leurs sels (y compris les sels de sodium et potassium), utilisés seuls ou en combinaison.

Le résidu alkyle en C1-C8 peut être linéaire ou ramifié, aliphatique ou aromatique. Il peut s'agir de résidu alkyl choisi dans le groupe consistant en méthyl, éthyl, propyl, isopropyl, butyl, isopropyl, pentyl, hexyl, octyl, benzyl.

Comme parabènes on peut citer les esters de l'acide parahydroxybenzoïque, tels que le méthylparabène (ou 4-hydroxybenzoate de méthyle), l'éthylparabène (ou 4-hydroxybenzoate d'éthyle), le propylparabène (ou 4-hydroxybenzoate de propyle), l'isopropylparabène, le butylparabène, l'isobutylparabène, le benzylparabène.

Les parabènes comprennent aussi les sels, en particulier les sels de sodium et de potassium.

En termes des parabènes ainsi à éviter et dont on cherche à limiter la présence ou la concentration on peut citer en particulier et d'une façon non-exhaustive le méthylparabène ou 4-hydroxybenzoate de méthyle (E218) et son sel de sodium (E219), l'éthylparabène ou 4-hydroxybenzoate d'éthyle (E214) et son sel de sodium (E215), le propylparabène ou 4-hydroxybenzoate de propyle (E216) et son sel de sodium (E217), le butylparabène et son sel de sodium.

Selon un mode de réalisation de la présente invention, on cherchera aussi à maintenir la concentration de conservateur choisi parmi le phénoxyethanol, l'hydroxyanisole butylé (ou BHA), le chlorocrésol, et les donneurs de formaldéhyde, utilisés seuls ou en combinaison en dessous d'un seuil de 0,01% en poids par rapport au poids total de la préparation, encore préférentiellement de 0,003% ou encore 0,001% en poids par rapport au poids total de la préparation. Encore préférentiellement, la composition selon la présente invention est également essentiellement dénuée, c'est-à-dire qu'elle contient une concentration de l'ordre de 0%, plus particulièrement inférieure à 0,01% en poids par rapport au poids total de la préparation, encore préférentiellement inférieure à 0,003% ou inférieure à 0,001% en poids par rapport au poids total de la préparation, en acide benzoïque ainsi que ses sels et/ou de benzyle alcool.

Dans un mode préférentiel, la composition de l'invention contient des conservateurs à l'exclusion de ceux du type parabène, mais aussi à l'exclusion du phénoxyethanol, à l'exclusion de l'hydroxyanisole butylé, à l'exclusion du chlorocrésol, à l'exclusion des donneurs de formaldéhyde. L'expression « à l'exclusion de », au même titre que l'expression « ne contient pas », signifie, au sens de la présente invention, que la composition est essentiellement dénuée des composants concernés. La concentration desdits composés pourra ainsi être de l'ordre de traces, c'est-à-dire une concentration de l'ordre de 0%, particulièrement inférieure à 0,01% en poids par rapport au poids de la préparation, encore préférentiellement pas plus que 0,003% ou pas plus que 0,001% en poids par rapport au poids total de la préparation.

Dans un mode encore préférentiel, la composition de l'invention ne contient aucun conservateur choisi parmi les parabènes, les benzoates, le benzyle alcool, le phénoxyethanol, le l'hydroxyanisole butylé, le chlorocrésol, seuls ou en combinaison, en particulier aucun de ces conservateurs à une concentration de plus que 0,01% en poids par rapport au poids de la préparation, encore préférentiellement pas plus que 0,003% ou pas plus que 0,001% en poids par rapport au poids total de la préparation.

Dans une de ses réalisations, la composition selon la présente invention est également essentiellement dénuée des donneurs de formaldéhyde, elle contient donc entre 0% et 0,01%, préférentiellement entre 0% et 0,003%, encore préférentiellement entre 0% et 0,001% des donneurs de formaldéhyde en poids par rapport au poids total de la préparation.

Selon une réalisation particulière, la composition selon la présente invention est également essentiellement dénuée de l'hydroxytoluène butylé ou BHT, elle contient donc entre 0% et 0,01%, préférentiellement entre 0% et 0,003%, encore préférentiellement entre 0% et 0,001% des de l'hydroxytoluène butylé en poids par rapport au poids total de la préparation.

Dans une de ses réalisations, la composition selon la présente invention est essentiellement dénuée de conservateur aromatique, elle contient donc entre 0% et 0,01%, préférentiellement entre 0% et 0,003%, encore préférentiellement entre 0% et 0,001% de conservateur aromatique en poids par rapport au poids total de la préparation.

Les termes « essentiellement dénué » ou « essentiellement dénuée » ainsi que « essentiellement libre », « essentiellement exempt(e) » et « essentiellement absent(s) » dans le sens de la présente invention indiquent que la substance concernée ou les substances concernées sont absentes de la composition en question, ou présent en tant que traces à un niveau aussi faible que pratiquement faisable ; en particulier à une concentration de l'ordre de 0%, particulièrement inférieure à 0,01% en poids par rapport au poids total de la préparation, encore préférentiellement inférieure à 0,003% ou inférieure à 0,001% en poids par rapport au poids total de la préparation. Encore préférentiellement, la concentration de la substance concernée dans la composition en question est en-dessous de la limite de détection par les méthodes d'analyse applicables utilisées habituellement par l'homme du métier.

Les compositions selon la présente invention contiennent un ou plusieurs conservateurs à l'exclusion des parabènes, pour assurer la stabilité microbiologique de la préparation. En vue de la population cible qui inclut les enfants et les nourrissons, et des adultes à peau sensible, la présente invention cherche à éviter ou de limiter les conservateurs irritants et/ou allergisants. Une autre façon complémentaire de s'adapter aux conditions d'une application sur peau sensible est de limiter la concentration des conservateurs utilisés. Certaines réalisations préférentielles de la présente invention contiennent des combinaisons de deux ou plusieurs conservateurs, particulièrement trois ou plus conservateurs, plus particulièrement encore 4 ou plus conservateurs, à l'exclusion des parabènes, permettant ainsi de répondre aux critères en matière d'efficacité de conservation antimicrobienne et de limiter en même temps la concentration de chacun des conservateurs grâce à leur interaction synergique.

Le au moins un conservateur, différent des parabènes, présent dans la composition selon l'invention est choisi dans le groupe consistant en, l'hexanediol, l'éthylhexylglycérine, le pentylèneglycol, le butylèneglycol, le 1,2 octanediol (caprylyl glycol) ainsi que leurs mélanges.

Le au moins un conservateur, différent des parabènes, sera présent aux concentrations adaptées pour assurer la stabilité microbiologique de la composition.

Avantageusement, elle contient un ou plusieurs conservateurs, différent des parabènes, choisi dans le groupe consistant en l'hexanediol, l'éthylhexylglycérine, le pentylèneglycol, et leurs mélanges, aux concentrations adaptées pour assurer la stabilité microbiologique de la composition.

Dans un mode de la composition selon l'invention, la composition selon l'invention contient de l'hexanediol. L'hexanediol peut être utilisé seul ou en combinaison avec au moins un autre conservateur. Dans un mode particulier de la composition, elle ne contient pas d'autre conservateur que hexanediol. Lorsqu'il est utilisé comme seul conservateur, l'hexanediol est présent dans la composition selon l'invention à une concentration de 3% à 10%, préférentiellement d'environ 5% en poids par rapport au poids total de la composition. Dans un autre mode de la composition selon l'invention, l'hexanediol est utilisé en combinaison avec au moins un autre conservateur, avec une concentration de hexanediol de 0,2% à 3%, préférentiellement de 0,3% à 2%, encore préférentiellement d'environ 0,4%, 0,45% ou 0,5% en poids par rapport au poids total de la composition.

Dans un mode de sa réalisation, la composition selon l'invention contient de l'éthylhexylglycérine à une concentration de 0,15% à 1%, préférentiellement de 0,2% à 0,6 %, encore préférentiellement d'environ 0,3%, d'environ 0,4% ou d'environ 0,5% en poids par rapport au poids total de la composition. L'éthylhexylglycérine peut être utilisé seul ou en combinaison avec un autre conservateur. Dans un mode préférentiel de la composition selon l'invention, l'éthylhexylglycérine est utilisé en combinaison avec au moins un autre conservateur. Dans un autre mode de la composition, elle ne contient pas d'autre conservateur que l'éthylhexylglycérine.

Selon un mode de réalisation particulier, la composition selon l'invention contient du pentylèneglycol. Le pentylèneglycol peut être utilisé seul ou en combinaison avec au moins un autre conservateur. Dans un mode particulier de la composition, elle ne contient pas d'autre conservateur que pentylèneglycol. Lorsqu'il est utilisé comme seul conservateur, le pentylèneglycol et présent dans la composition selon l'invention à une concentration de 4% à 10%, préférentiellement d'environ 5% en poids par rapport au poids total de la composition. Dans un autre mode de la composition selon l'invention, le pentylèneglycol est utilisé en combinaison avec au moins un autre conservateur, avec une concentration de pentylèneglycol de 0,3% à 3%, préférentiellement de 0,3% à 0,5%, encore préférentiellement d'environ 0,3%, d'environ 0,4% ou d'environ 0,45% en poids par rapport au poids total de la composition.

Dans un mode de réalisation, la composition selon l'invention contient du butylèneglycol. Préférentiellement, le butylèneglycol est utilisé en combinaison avec au moins un autre conservateur. Au sein d'un mode de la composition selon l'invention, le butylèneglycol est utilisé à une concentration de 1% à 10%, préférentiellement de 2% à 5%, encore préférentiellement à environ 4% ou environ 4,4% en poids par rapport au poids total de la composition.

Dans un autre mode de réalisation de la composition selon l'invention, elle contient du 1,2 octanediol (caprylyl glycol) à une concentration de 0,1% à 1%, préférentiellement d'environ 0,3%, d'environ 0,4% ou d'environ 0,5% en poids par rapport au poids total de la composition. Préférentiellement, le 1,2 octanediol (caprylyl glycol) est utilisé en combinaison avec au moins un autre conservateur.

Selon un mode de réalisation particulier, la composition selon l'invention contient de l'éthylhexylglycérine à une concentration de 0,15% à 1%, préférentiellement de 0,2% à 0,6%, encore préférentiellement d'environ 0,3%, d'environ 0,4% ou d'environ 0,5% en poids par rapport au poids total de la composition et du pentylèneglycol à une concentration de 0,1% à 3%, préférentiellement de 0,3% à 0,5%, encore préférentiellement d'environ 0,3%, d'environ 0,4% ou d'environ 0,45% en poids par rapport au poids total de la composition, et, encore avantageusement, elle ne contient pas d'autre conservateur que éthylhexylglycérine et pentylèneglycol. Dans une telle composition, lesdits conservateurs pourront être avantageusement associés à un mélange de deux gélifiants, ledit mélange de gélifiants comprenant, ou consistant en, un premier gélifiant de type polyacrylamide étant le copolymère d'acrylamide/sodium acryloyldiméthyltaurate avec isohexadecane et polysorbate en quantité d'environ 0,5%, ou d'environ 0,6%, en poids par rapport au poids total de la composition ; un second gélifiant étant un carbomère, en particulier le carbomère 980, en quantité d'environ 0,025 %, ou d'environ 0,05%, en poids par rapport au poids total de la composition.

Dans un mode de réalisation, la composition selon l'invention contient de l'éthylhexylglycérine à une concentration de 0,15% à 1%, préférentiellement de 0,2% à 0,5%, encore préférentiellement de 0,3% à 0,5% en poids par rapport au poids total de la composition, et du butylèneglycol à une concentration de 1% à 10%, préférentiellement de 2% à 4,4% en poids par rapport au poids total de la composition, et, encore avantageusement, elle ne contient pas d'autre conservateur que éthylhexylglycérine et butylèneglycol.

Dans un autre mode de réalisation, la composition selon l'invention contient de l'éthylhexylglycérine à une concentration de 0,15% à 1%, préférentiellement de 0,2% à 0,5%, encore préférentiellement de 0,3% à 0,5% en poids par rapport au poids total de la composition, et du 1,2 octanediol (caprylyl glycol) à une concentration de 0,1% à 1%, préférentiellement de 0,3% en poids par rapport au poids total de la composition, et, encore avantageusement, elle ne contient pas d'autre conservateur que éthylhexylglycérine et 1,2 octanediol (caprylyl glycol).

La composition selon l'invention contient au moins deux agents gélifiants.

Un des agents gélifiants est de type polyacrylamide. Le polyacrylamide peut être un homopolymère ou un copolymère d'acrylamide avec d'autres monomères. Cet autre monomère peut en particulier être l'acryloyldiméthyltaurate, l'acide (meth)acrylique, les esters de l'acide (meth)arylique et leurs mélanges. En particulier le polyacrylamide peut être un copolymère acrylamide/acryloyldiméthyltaurate. Le comonomère peut être sous forme acide ou neutralisée avec un agent alcalin ou alcalino-terreux.

Un second agent gélifiant est choisi dans le groupe consistant en : gomme de xanthane, carbomère 980, carbomère 974, hydroxyéthylcelllulose, hydroxypropylméthylcellulose et leurs mélanges.Outre le premier et second agent gélifiant, des exemples d'agents gélifiants appropriés contiennent (d'une façon non-limitante) les polymères stabilisants tels que la gomme xanthane, la gomme gellane (E4183), les carbomères (polymères synthétiques d'acide acrylique) tels que par exemple le carbomère 974 ou le carbomère 980, l'hydroxyethylcelllulose (par exemple l'hypromellose 2910), l'hydroxypropylméthylcellulose (HPMC), la carboxyméthylcellulose. En particulier les au moins deux gélifiants de la composition selon l'invention comprennent un gélifiant de type polymère polyacrylamide.

Comme gélifiant de la famille des polyacrylamides on peut citer le mélange copolymer acrylamide/ Sodium acryloyldimethyltaurate / isohexadecane / polysorbate 80 vendu par la société SEPPIC, le mélange polyacrylamide/isoparaffine C13-14/laureth-7 vendu par la société SEPPIC (à titre d'exemple, on peut citer les produits de la société Seppic de la gamme Sepineo^{®}, Sepigel^{®} ou Simulgel^{®}). Le mélange copolymère acrylamide/sodium acryloyldiméthyltaurate avec isohexadecane et polysorbate-(aussi appelé mélange copolymère acrylamide/sodium acryloyldiméthyltaurate ou encore Copolymer Acrylate / Sodium Acryloydimethyl taurate & Isohexadecane & Polysorbate 80) sera désigné dans les exemples par « mélange copolymère acrylamide ».

A titre d'exemple non limitatif d'agents gélifiants on peut citer les carbomers vendus sous le nom d'Ultrez 20^{®}, d'Ultrez 10^{®}, de Carbopol 1382^{®} ou de Carbopol ETD2020NF^{®}, de Carbopol 981 ou encore Carbopol 980 par la Société Lubrizol, les polysaccharides avec à titre d'exemples non limitatifs la gomme de xanthane telle que le Xanturall80^{®} vendu par la société Kelco, la gomme gellane vendue sous le nom de Kelcogel par la société Kelco, la gomme guar, la cellulose et ses dérivés tel que la cellulose microcristalline et carboxymethyl cellulose de sodium vendue sous le nom d' Avicel CL-611 par la société FMC Biopolymer, l'hydroxypropylmethylcellulose en particulier le produit vendu sous le nom de Methocel E4M premium par la société Dow Chemical ou l'hydroxyéthylcellulose , en particulier, le produit vendu sous le nom de Natrosol HHX 250^{®} par la société Ashland, la carboxymethylcellulose sodique , en particulier la gomme de cellulose Blanose 7F vendue par la société Ashland, la famille des aluminium magnésium silicates tel que le Veegum K vendu par la société Vanderbilt, la famille des amidons modifiés tels que l'amidon de pomme de terre modifié vendu sous le nom de Structure Solanace ou bien leurs mélanges, la famille des carraghénanes en particulier réparties sous quatre grandes familles : κ, λ, β, ω tel que les Viscarin^{®} et les Gelcarin^{®} commercialisés par la société IMCD et leurs mélanges.

Les carbomères sont des polymères synthétiques hydrophiles de l'acide acrylique, de haut poids moléculaire. Les carbomères sont produits par polymérisation de l'acide acrylique pour former des polymères d'acide acrylique (acide polyacrylique) réticulés, de haut poids moléculaire, puis éventuellement réticulés par exemple avec des éthers de pentaérythritol. Il peut s'agir d'homopolymères d'acide acrylique linéaires ou réticulés avec un un réticulant tel qu'un allyléther de pentaérythritol, un allyléther de saccharose ou un allyléther de propylène (on peut citer les produits CArbopol^{®} de Lubrizol par exemple).

Carbomère est le nom générique d'une classe de molécules absorbant l'eau en quantité très importante. Les carbomères sont des polymères réticulés d'acide acrylique de masse moléculaire élevée qui, une fois neutralisés, ont la capacité d'absorber et de retenir l'eau, ce qui donne un gel. La plupart des carbomères sont classés comme ayant une rhéologie longue ou courte pour indiquer la nature du polymère et le taux de réticulation. Une rhéologie courte correspond à un polymère fortement réticulé, tandis qu'une rhéologie longue correspond à un polymère légèrement réticulé.

Dans le cas de la présente invention on préférera les carbomères réticulés, en particulier fortement réticulés.

On préfèrera en particulier le Carbomère 980 ou le carbomère 974.

Dans un mode de réalisation, la composition selon l'invention contient entre 0,1 et 0,4% de gomme de Xanthane, de préférence environ 0,1% en poids par rapport au poids total de la composition ou 0,2% en poids par rapport au poids total de la composition.

Avantageusement, la composition selon l'invention présente entre 0,2 et 2% de mélange gélifiant de type polyacrylamide, de préférence entre 0,3% et 1%, et encore préférentiellement environ 0,5% en poids par rapport au poids total de la composition. Dans un autre mode de la réalisation, elle comprend environ 0,6% de mélange gélifiant de type polyacrylamide en poids par rapport au poids total de la composition. Dans encore un autre mode de la réalisation, elle comprend environ 0,75% de mélange gélifiant de type polyacrylamide en poids par rapport au poids total de la composition.

Encore avantageusement, le mélange gélifiant de type polyacrylamide de la composition est le mélange copolymère acrylamide/sodium acryloyldiméthyltaurate avec isohexadecane et polysorbate. Préférentiellement, la composition selon l'invention présente donc entre 0,2 et 2% du mélange copolymère acrylamide/sodium acryloyldiméthyltaurate avec isohexadecane et polysorbate, de préférence entre 0,5% et 1%, et encore préférentiellement environ 0,5% en poids par rapport au poids total de la composition. Dans un autre mode de la réalisation, elle comprend environ 0,6% de mélange copolymère acrylamide/sodium acryloyldiméthyltaurate avec isohexadecane et polysorbate en poids par rapport au poids total de la composition. Dans encore un autre mode de la réalisation, elle comprend environ 0,75% de mélange copolymère acrylamide/sodium acryloyldiméthyltaurate avec isohexadecane et polysorbate en poids par rapport au poids total de la composition.

Dans un mode préférentiel, la composition selon l'invention présente entre 0,2 et 2% de mélange gélifiant de type polyacrylamide, avantageusement le mélange copolymère acrylamide/sodium acryloyldiméthyltaurate avec isohexadecane et polysorbate, de préférence entre 0,5% et 1%, et encore préférentiellement environ 0,5% ou 0,6% en poids par rapport au poids total de la composition en combinaison avec au moins un autre agent gélifiant choisi dans le groupe comprenant la gomme de xanthane, le carbomère 980, le carbomère 974, l'hydroxyéthylcelllulose (hypromellose 2910), l'hydroxypropylméthylcellulose (HPMC), utilisés seuls ou en mélange. Préférentiellement, l'autre agent gélifiant est le carbomère 980.

Avantageusement, la composition selon l'invention présente entre 0,01 et 0,5% de carbomère 980, de préférence entre 0,025% et 0,1%, de préférence environ 0,025%, environ 0,03% ou environ 0,05% en poids par rapport au poids total de la composition.

Selon une réalisation préférentielle, la composition selon la présente invention contient à la fois le mélange copolymère acrylamide/sodium acryloyldiméthyltaurate avec isohexadecane et polysorbate ; et un carbomère, en particulier le carbomère 980, avec une concentration de mélange copolymère acrylamide/sodium acryloyldiméthyltaurate avec isohexadecane et polysorbate de préférence entre 0,5% et 1%, et encore préférentiellement de environ 0,5% en poids par rapport au poids total de la composition ou de environ 0,6% en poids par rapport au poids total de la composition ; et une concentration de carbomère, en particulier le Carbomère 980, de préférence entre 0,025% et 0,1%, encore préférentiellement environ 0,025%, environ 0,03% ou environ 0,05% en poids par rapport au poids total de la composition. De manière préférée, la composition selon l'invention comprend deux conservateurs, autres que parabènes, consistant en éthylhéxylglycérine et pentylèneglycol ; en association avec un mélange de deux gélifiants, ledit mélange de gélifiants comprenant ou consistant en :
- un gélifiant de type copolymère polyacrylamide étant l'acrylamide/sodium acryloyldiméthyltaurate avec isohexadecane et polysorbate en quantité d'environ 0,2 à 1%, particulièrement 0,3 à 0,7% en poids par rapport au poids total de la composition ;
- un second gélifiant étant un carbomère, en particulier le carbomère 980, en quantité d'environ 0,01 à 0,1%, particulièrement entre 0,02 à 0,05% en poids par rapport au poids total de la composition.

La concentration des agents gélifiants est choisie en fonction de la consistance de la composition finale. Cette consistance cible peut être mesurée, à température ambiante, à la date de fabrication (T0) et/ou après stockage pour 3 mois ou 6 mois, voire 12 mois. Dans un mode particulier, la consistance peut être mesurée, à température ambiante, après un stockage de la composition à 40°C et 25% d'humidité relative pour 3 mois ou 6 mois.

Avantageusement, la concentration de l'agent gélifiant ou des agents gélifiants est donc choisie d'une façon qui permet d'obtenir une composition selon l'invention d'une consistance à fabrication (T0) comprise entre 70 et 230 g à température ambiante et pression atmosphérique de 760 mm Hg, d'une manière préférée entre 80 et 220 g et d'une manière encore préférée entre 90 et 210 g.

La mesure de consistance est donnée pour une température ambiante d'environ 20°C et sous une pression atmosphérique de 760 mm Hg environ. La consistance est mesurée selon les modalités bien connues d'un homme du métier. On peut citer plusieurs manières.

La mesure de consistance est une mesure texturométrique ou pénétrométrique d'un produit d'une substance, selon laquelle on place un échantillon du dit produit de ladite substance dans un support, on fait pénétrer du haut vers le bas une sonde calibrée dans cet échantillon, selon une course à accomplir dans un volume la masse de 1 d'échantillon et selon une vitesse préalablement choisie. Les dispositifs connus, adaptés à cette méthode de mesure, appelée analyse de texture consistométrie ou texturométrie ou pénétrométrie ont comme principe commun de faire pénétrer une sonde d'un calibre et/ou d'un poids déterminé, par exemple un cylindre ou un cône renversé, dans un volume la masse de l'échantillon. Ces systèmes peuvent se fonder sur la profondeur de pénétration atteinte en un temps donné, ou sur le temps mis par la sonde pour effectuer un certain trajet, ou encore sur la force, en grammes, à exercer pour que la sonde accomplisse un certain trajet en un certain temps, à une température déterminée. Dans les analyseurs de texture pénétromètres commercialisés, un moteur fait descendre une sonde, fixée à un support mobile en translation verticale, dans l'échantillon, qui est placé sur un plateau fixe. Un capteur de force solidaire de la sonde et/ou de son support mobile mesure la réaction de l'échantillon à la pénétration de la sonde, c'est-à-dire une force de réaction dirigée vers le haut et exprimée en grammes.

Dans un autre mode de l'invention, la concentration de l'agent gélifiant ou des agents gélifiants est choisi d'une façon qu'elle permet d'obtenir une composition selon l'invention avec une valeur de consistance comprise entre 40 et 210 g à température ambiante , de préférence entre 50 et 200 g, encore avantageusement entre 60 et 190 g quand cette valeur de consistance est déterminée après un stockage à 40°C et 25% d'humidité relative pendant 3 mois, encore préférentiellement aussi après un stockage pendant 6 mois.

Encore avantageusement, la concentration de l'agent gélifiant ou des agents gélifiants est choisi d'une façon qu'elle permet d'obtenir une composition selon l'invention d'une consistance comprise entre 70 et 230 g à température ambiante, d'une manière préférée entre 80 et 220 g et d'une manière encore préférée entre 90 et 210 g à fabrication, et d'une consistance comprise entre 40 et 210 g, de préférence entre 50 et 200 g, encore avantageusement entre 60 et 190 g après un stockage à 40°C et 25% d'humidité relative pendant 3 mois, encore préférentiellement aussi après un stockage pendant 6 mois.

Selon une réalisation préférentielle, la composition selon la présente invention contient à la fois un mélange gélifiant de type polyacrylamide, avantageusement le mélange copolymère acrylamide/sodium acryloyldiméthyltaurate avec isohexadecane et polysorbate ; et un second gélifiant, le carbomère 980. La concentration de mélange gélifiant de type polyacrylamide est de préférence entre 0,5% et 1%, et encore préférentiellement d'environ 0,5% en poids par rapport au poids total de la composition ou d'environ 0,6% en poids par rapport au poids total de la composition. La concentration de carbomère 980 est de préférence entre 0,01 et 0,1 %, particulièrement entre 0,025% et 0,1%, encore préférentiellement d'environ 0,025%, d'environ 0,03% ou d'environ 0,05% en poids par rapport au poids total de la composition, et la valeur de consistance de la composition à fabrication est comprise entre 70 et 230 g à température ambiante, d'une manière préférée entre 80 et 220 g et d'une manière encore préférée entre 90 et 210 g.

Selon une autre réalisation préférentielle, la composition selon la présente invention contient à la fois le mélange copolymère acrylamide/sodium acryloyldiméthyltaurate avec isohexadecane et polysorbate ; et un second gélifiant, le carbomère 980. La concentration du mélange copolymère acrylamide/sodium acryloyldiméthyltaurate avec isohexadecane et polysorbate est de préférence entre 0,2% et 1%, et encore préférentiellement entre 0,3 et 0,7%, plus particulièrement d'environ 0,5% en poids par rapport au poids total de la composition ou encore d'environ 0,6% en poids par rapport au poids total de la composition. La concentration de carbomère 980 de préférence entre 0,01% et 0,1%, en particulière entre 0,02 et 0,05%, encore préférentiellement d'environ 0,025%, d'environ 0,03% ou d'environ 0,05% en poids par rapport au poids total de la composition, et la valeur de consistance de la composition après un stockage à 40°C et 25% d'humidité relative pendant 3 mois est comprise entre 40 et 210 g à température ambiante, de préférence entre 50 et 200 g, encore avantageusement entre 60 et 190 g ; encore préférentiellement, ces valeurs de consistance sont également maintenues après un stockage pendant 6 mois à 40°C et 25% d'humidité relative.

Encore préférentiellement, la composition selon la présente invention contient à la fois le mélange copolymère acrylamide/sodium acryloyldiméthyltaurate avec isohexadecane et polysorbate, et le second gélifiant, le carbomère 980. La concentration de mélange copolymère acrylamide/sodium acryloyldiméthyltaurate avec isohexadecane et polysorbate est de préférence entre 0,5% et 1%, et encore préférentiellement de environ 0,5% en poids par rapport au poids total de la composition ou de environ 0,6% en poids par rapport au poids total de la composition. La concentration de carbomère 980 est de préférence entre 0,025% et 0,1%, encore préférentiellement d'environ 0,025%, d'environ 0,03% ou d'environ 0,05% en poids par rapport au poids total de la composition, et la valeur de consistance de la composition à fabrication est comprise entre 70 et 230 g à température ambiante, d'une manière préférée entre 80 et 220 g et d'une manière encore préférée entre 90 et 210 g, la valeur de consistance de la composition après un stockage à 40°C et 25% d'humidité relative pendant 3 mois est comprise entre 40 et 210 g à température ambiante, de préférence entre 50 et 200 g, encore avantageusement entre 60 et 190 g, et, optionnellement, la valeur de consistance de la composition après un stockage à 40°C et 25% d'humidité relative pendant 6 mois est également comprise entre 40 et 210 g à température ambiante, de préférence entre 50 et 200 g, encore avantageusement entre 60 et 190 g.

Avantageusement, la composition selon l'invention comprend entre 10 et 20%, préférentiellement entre 13 et 17%, et de manière particulièrement préférée d'environ 15% de glycérol, entre 3 et 20%, préférentiellement entre 5 et 10% et de manière particulièrement préférée environ 8% de vaseline, entre 0,5 et 5%, préférentiellement entre 1 et 3% et de manière particulièrement préférée environ 2% de paraffine liquide en poids par rapport au poids total de la composition, de l'éthylhexylglycérine à une concentration de 0,15% à 1%, préférentiellement de 0,2% à 0,5%, encore préférentiellement d'environ 0,3%, d'environ 0,4% ou d'environ 0,5% en poids par rapport au poids total de la composition, du pentylèneglycol à une concentration de 0,3% à 3%, préférentiellement de 0,3% à 0,45%, encore préférentiellement d'environ 0,3%, d'environ 0,4% ou d'environ 0,45% en poids par rapport au poids total de la composition, et, encore avantageusement, elle ne contient pas d'autre conservateur que éthylhexylglycérine et pentylèneglycol, en particulier pas de parabènes. De préférence, ladite composition contient en même temps à la fois le mélange copolymère acrylamide/sodium acryloyldiméthyltaurate avec isohexadecane et polysorbate en association avec le carbomère 980, avec une concentration de mélange copolymère acrylamide/sodium acryloyldiméthyltaurate avec isohexadecane et polysorbate de préférence entre 0,5% et 1%, et encore préférentiellement de environ 0,5% en poids par rapport au poids total de la composition ou de environ 0,6% en poids par rapport au poids total de la composition, une concentration de Carbomère, en particulier carbomère 980, de préférence entre 0,025% et 0,1%, encore préférentiellement environ 0,025%, environ 0,03% ou environ 0,05% en poids par rapport au poids total de la composition.

La composition selon l'invention comprend, en outre, des ingrédients usuels dermatologiquement et/ou cosmétiquement compatibles. Un ingrédient dermatologiquement et/ou cosmétiquement compatible peut être tout ingrédient parmi ceux connus de l'homme de l'art en vue d'obtenir une composition pour l'application topique sous forme de crème, d'une lotion, d'un gel, d'une pommade, d'une émulsion, d'une microémulsion, d'un spray, etc.

Ainsi, la composition selon l'invention peut en outre contenir des additifs et aides à la formulation, tels que des émulsionnants, des épaississants, des fixateurs d'eau, des agents d'étalement, des stabilisants. L'homme de l'art adaptera le choix de ces additifs en fonction de l'effet attendu.

Ainsi, la composition selon l'invention peut comprendre en outre un ou plusieurs composés choisis dans le groupe constitué des parfums, colorants, vitamines, correcteurs de pH.

Selon une réalisation préférentielle, la composition selon la présente invention ne contient pas de parfum.

Des émulsionnants appropriés comprennent l'acide stéarique, la trolamine, le PEG-40-stéarate.

Avantageusement, la composition selon l'invention comprend un ou plusieurs émulsionnants.

Avantageusement, la composition selon l'invention présente entre 1 et 5% d'acide stéarique, de préférence environ 3% en poids par rapport au poids total de la composition.

Avantageusement, la composition selon l'invention présente entre 0,2 et 2% de trolamine, de préférence environ 0,5% en poids par rapport au poids total de la composition.

Avantageusement, la composition selon l'invention présente entre 0 et 2% de PEG-40-stéarate, de préférence environ 0,5% en poids par rapport au poids total de la composition.

Des épaississants appropriés comprennent le monostéarate de glycérol, les PEG (polyéthylène glycol), en particulier le PEG 600.

Avantageusement, la composition selon l'invention comprend un ou plusieurs épaississants.

Avantageusement, la composition selon l'invention présente entre 2 et 10% de monostéarate de glycérol, de préférence environ 5% en poids par rapport au poids total de la composition.

Avantageusement, la composition selon l'invention présente entre 2 et 10% de polyéthylène glycol, de préférence environ 5% en poids par rapport au poids total de la composition. Préférentiellement, il s'agit de polyéthylène glycol 600.

Avantageusement, la composition selon l'invention présente entre 2 et 10% de PEG 600, de préférence environ 5% en poids par rapport au poids total de la composition.

De préférence, la composition selon l'invention contient un ou plusieurs agents d'étalement. Des agents d'étalement appropriés comprennent la diméthicone, le polydiméthylcyclosiloxane, le myristate d'isopropyle, le palmitate d'isopropyle, l'isononanoate de cetostéaryle, l'oléate de décyle, l'oléate d'éthyle.

Avantageusement, la composition selon l'invention présente entre 0,2 et 2% de diméthicone, de préférence environ 0,5% de diméthicone en poids par rapport au poids total de la composition.

Dans un mode de l'invention, la composition selon l'invention présente entre 1 et 3% de polydiméthylcyclosiloxane, de préférence environ 2,5% en poids par rapport au poids total de la composition. Dans un autre mode d'invention, la composition selon l'invention contient moins que 0,1% de polydiméthylcyclosiloxane, avantageusement elle ne contient pas de polydiméthylcyclosiloxane.

Avantageusement, la composition selon l'invention présente entre 0,5 et 2,5% de myristate d'isopropyle, de préférence environ 1% en poids par rapport au poids total de la composition.

Avantageusement, la composition selon l'invention présente entre 0, 5 et 2,5% de palmitate d'isopropyle, de préférence environ 1% en poids par rapport au poids total de la composition.

Avantageusement, la composition selon l'invention se présente sous forme d'émulsion blanche. Préférentiellement, elle ne contient pas de colorant, ce qui comprend qu'il n'y a pas de colorant qui est ajouté lors de la fabrication de la composition. Avantageusement, la composition selon l'invention a une odeur convenable, de préférence d'une évocation neutre. Préférentiellement, elle ne contient pas de parfum, ce qui comprend qu'il n'y a pas de parfum qui est ajouté lors de la fabrication de la composition.

D'un point de vue d'une utilisation confortable, l'émulsion à utilisation topique dans le sens de la présente invention doit présenter une texture agréable, un aspect homogène et une odeur appropriée.

En termes de critères physicochimiques, on note notamment une bonne consistance et viscosité de la préparation et la granulométrie (la taille des globules) ainsi qu'une fourchette de pH établi pour l'utilisation cutanée des émulsions en question. L'homme du métier connaît des méthodes établies pour une quantification de ces paramètres.

Avantageusement, la composition selon l'invention présente un pH (mesuré au 1/10 dans l'eau) légèrement basique, d'une valeur compris entre pH 7,1 et pH 8,9, préférentiellement entre pH 7,4 et pH 8,7.

Avantageusement, au moins 95% des globules au sein de la préparation présentent une taille inférieure à 30µm, encore avantageusement inférieure à 25µm.

L'aptitude à l'étalement est approchée par des mesures de consistance, conduites à l'aide d'un analyseur de texture, elle est exprimée en g, à température ambiante. La consistance recherchée doit permettre un bon étalement du produit sur la peau et doit être donc suffisamment élevée à la fabrication (T0) pour assurer une valeur de consistance convenable toute la durée de vie du produit. Une valeur acceptable à péremption est d'environ 40 g à température ambiante, valeur en dessous de laquelle l'émulsion devient trop fluide pour présenter une préhension (et un pouvoir filmogène) acceptable.

La consistance de la composition selon l'invention à fabrication (T0) est de préférence comprise entre 70 et 230 g à température ambiante, d'une manière préférée entre 80 et 220 g et d'une manière encore préférée entre 90 et 210 g. Compte tenu d'une certaine chute possible (et tolérable) de la consistance d'une crème au cours du temps tel que bien connu par l'homme du métier, la consistance de la composition après stockage pour 3 mois à température ambiante est de préférence comprise entre 40 et 210 g à température ambiante, d'une manière préférée entre 50 et 200g et d'une manière encore préférée entre 60 et 190 g. Encore préférentiellement, ces valeurs sont mesurées également après un stockage à 40°C et 25% d'humidité relative pour 6 mois.

La viscosité de la composition selon l'invention à fabrication est de préférence comprise entre 7000 mPa·s et 40000 mPa·s à température ambiante, d'une manière préférée entre 9000 mPa·s et 30000 mPa·s. Après stockage pour 3 mois à 40°C et 25% d'humidité relative la valeur de viscosité est de préférence comprise entre 6000 mPa·s et 30000 mPa·s à température ambiante, d'une manière préférée entre 7000 mPa·s et 25000 mPa·s. Encore préférentiellement, ces valeurs sont mesurées également après un stockage à 6 mois à 40°C et 25% d'humidité relative.

La mesure de viscosité consiste à mesurer les paramètres de cisaillement du matériau dans les rhéomètres ou viscosimètres à mobile tournant. L'échantillon est placé dans un godet formant le stator, dans lequel on engage un rotor qui peut avoir diverses formes selon le type d'appareil. Le rotor est mis en mouvement grâce à un moteur ; plus la viscosité est importante, plus le rotor est freiné. On lit sur une échelle de viscosités la valeur d'une résistance provoquée par la viscosité du matériau situé dans l'entrefer entre rotor et stator.

Dans un aspect particulier de l'invention, les compositions selon l'invention sont stables dans le temps. Les paramètres établis pour la composition selon l'invention doivent être maintenues dans le temps, dans le sens qu'une dégradation de la préparation est à éviter.

Des critères par rapport à la stabilité microbiologique des préparations à utilisation topique sont à consulter dans la Pharmacopée Européenne, avec des valeurs définies pour plusieurs types d'microorganismes, notamment les bactéries ainsi que les levures et champignons. Des critères microbiologiques sont par exemple définis dans la pharmacopée européenne 8ème édition (2016) §5.1.3 pour les produits topiques applicables par voie cutanée.

La stabilité des compositions selon l'invention peut être évaluée également par le maintien de l'aspect visuel comme jugé par inspection après stockage sous les conditions comme définies ; un déphasage du produit et une précipitation des ingrédients sont à éviter. Dans un autre aspect, cette stabilité concerne également l'absence des altérations importantes des caractéristiques olfactives au cours du temps, évaluée par sensation olfactive après stockage sous les conditions comme définies.

Un effet recherché dans un mode préférentiel de l'invention est l'obtention d'une émulsion stable à la chaleur au cours du stockage. Une dégradation des caractères organoleptiques de la préparation est donc à éviter, même sous condition de stockage à température élevée. Avantageusement, la composition selon l'invention ne présente pas de déphasage (jugée par inspection) 3 ou 6 mois après fabrication, ou même 8 ou 12 mois après fabrication, préférentiellement même sous conditions d'un stockage à température élevée, par exemple à 40°C ou même à 50°C. De même, une précipitation des composés (jugée par inspection) de l'émulsion est à éviter, préférentiellement également sous conditions de stockage à 40°C ou 50°C pendant 3 ou 6 mois, ou même 8 ou 12 mois.

Même si une certaine variabilité au cours de temps notamment dans les paramètres de la consistance et de la viscosité d'une émulsion pâteuse est bien connue et acceptable, une altération trop importante de la consistance et de la viscosité pendant le stockage de ces compositions est à éviter.

Selon un mode de réalisation de la présente invention, la composition selon l'invention est stable dans le temps. Par l'expression « stable dans le temps », on entend que les valeurs de consistance, à température ambiante, après stockage ne varient pas d'une façon trop importante par rapport aux valeurs à fabrication (T0) ; préférentiellement, la valeur de consistance, à température ambiante, d'une composition après un stockage à 40°C et 25% d'humidité relative pour 3 mois, voire 6 mois, ne diffère pas de plus de 50%, encore préférentiellement pas plus de 40%, et encore plus particulièrement pas plus de 30%, par rapport à la valeur de consistance à température ambiante de ladite composition à T0. Préférentiellement, la composition selon l'invention présente cependant une valeur de consistance comprise entre 40 et 210 g à température ambiante, d'une manière préférée entre 50 et 200g et d'une manière encore préférée entre 60 et 190 g après un stockage à 40°C et 25% d'humidité relative pour 3 mois, voire 6 mois.

Dans un autre mode de réalisation de la présente invention, les valeurs de viscosité après stockage ne doivent pas varier d'une façon trop importante par rapport aux valeurs à fabrication (T0) ; aussi par l'expression « stable dans le temps », on entend que, préférentiellement, la valeur de viscosité, à température ambiante, d'une composition après un stockage à 40°C et 25% d'humidité relative pour 3 mois, voire 6 mois, ne diffère pas de plus de 50%, particulièrement pas plus de 40%, par rapport à la composition à T0. Préférentiellement, la composition selon l'invention présente cependant une valeur de viscosité comprise entre 6000 mPa·s et 30000 mPa·s à température ambiante, d'une manière préférée entre 7000 mPa·s et 25000 mPa·s, après un stockage à 40°C et 25% d'humidité relative pour 3 mois, voire 6 mois.

L'expression « stable dans le temps » signifie que la consistance ne varie pas comme indiqué ci-dessus.

L'expression « stable dans le temps » signifie que la viscosité ne varie pas comme indiqué ci-dessus.

L'expression « stable dans le temps » signifie que la consistance ou la viscosité ne varient pas comme indiqué ci-dessus.

L'expression « stable dans le temps » signifie que la consistance et la viscosité ne varient pas comme indiqué ci-dessus.

La composition selon l'invention peut être une composition pharmaceutique ou une composition cosmétique. Elle est destinée à une utilisation topique.

La composition selon l'invention est considérée comme un émollient.

Un émollient selon l'invention est une composition ayant pour propriétés d'assouplir et de détendre les tissus de l'organisme, en l'occurrence la peau.

La présente description divulgue l'utilisation de la composition dans le traitement des peaux sèches, les états de sécheresse cutanée, en particulier chez les nourrissons ou les personnes âgées.

La composition selon l'invention est utile à toute indication dans la xérose et à toute population pour laquelle un émollient peut être utilisé.

La présente invention a également pour objet l'utilisation de la composition selon l'invention en tant qu'émollient.

La composition selon la présente invention est particulièrement adaptée à la pédiatrie et notamment chez les nourrissons.

La composition selon la présente invention est également particulièrement adaptée aux personnes âgées, notamment pour la xérose sénile ou asthéatotique.

La présente invention a également pour objet l'utilisation cosmétique de la composition selon l'invention, en particulier chez les nourrissons ou les personnes âgées, de préférence pour prévenir les états de sécheresse cutanée.

La présente invention a également pour objet la composition selon l'invention pour son utilisation dans la prévention ou le traitement des xéroses iatrogènes et autres effets secondaires de traitements nécessitant l'application d'un émollient.

Selon un autre mode de réalisation, la présente invention a également pour objet une composition selon l'invention pour son utilisation dans la prévention ou le traitement des signes et symptômes des états de sécheresse cutanée (xérose), notamment dans le cadre de certaines dermatoses (dermatite).

La présente invention a également pour objet une composition selon l'invention pour son utilisation dans la prévention ou le traitement des brûlures superficielles de faibles étendues.

La présente invention a également pour objet une composition selon l'invention pour son utilisation dans la prévention ou le traitement des poussées eczémateuses observées chez les patients atteints de dermatite atopique.

L'invention vise ainsi une composition selon l'invention, pour son utilisation en tant que dispositif médical.

L'invention vise encore une composition selon l'invention, pour son utilisation dans la prévention et/ou le traitement des xéroses iatrogènes et autres effets secondaires de traitements nécessitant l'application d'un émollient.

L'invention vise aussi une composition selon l'invention, pour son utilisation dans la prévention et/ou le traitement des xéroses apparues comme effets secondaires ou symptômes cutanés de pathologies telles que l'insuffisance rénale ou diabétique.

C'est un objet que de fournir une composition selon l'invention pour son utilisation dans la prévention et/ou le traitement des états de sécheresse cutanée associés à certaines dermatoses telles que dermatite atopique, états ichtyosiques, psoriasis.

C'est un objet que de fournir une composition selon l'invention pour son utilisation pour diminuer la fréquence et/ou réduire l'intensité des poussées eczémateuses observées chez les patients atteints de dermatite atopique.

C'est un objet que de fournir une composition selon l'invention pour son utilisation pour son utilisation dans la prévention et/ou le traitement des brûlures superficielles.

C'est un objet que de viser une utilisation cosmétique d'une composition selon l'invention, en particulier chez les nourrissons ou les personnes âgées.

C'est un objet que de viser une utilisation cosmétique d'une composition selon l'invention pour prévenir les états de sécheresse cutanée.

Partie expérimentale : Les exemples suivants illustrent l'invention qui est limitée par les revendications.

### Exemple 1) Criblage conservateurs

Dans un premier temps, un criblage des conservateurs est effectué. Le but est de déterminer si les conservateurs qui sont incluses dans la formule assurent une protection contre une contamination microbiologique. Les critères appliqués pour cette évaluation sont celles de la pharmacopée européenne, 8^{ème} édition (2016), paragraphe §5.1.3, comme détaillé plus bas. Une première évaluation de la compatibilité des conservateurs en vue des autres aspects (par exemple odeur, aspect, potentiel irritant ou allergisant) est également effectuée.

Tous les conservateurs sont testés dans une formulation de la composition suivante :
15 % de glycérol,
8% de vaseline,
2% de paraffine liquide
entre 3% et 4% d'émulsionnants (3% d'acide stéarique + <1% TEA ou TRIS)
5% de monostéarate de glycérol,
5% de polyéthylèneglycol,
agents d'étalement,
qsp 100% en eau purifiée.

Incorporation des conservateurs à chaud, dans la phase aqueuse ou dans la phase grasse, selon les solubilités des conservateurs.

### Test microbiologique :

Les formulations sont volontairement contaminées par environ 10⁶ germes/mL parmi les souches *Pseudomonas aeruginosa* (PA), *Staphylocoque aureus* (SA), *Escherichia coli* (EC), *Candida albicans* (CA), *Aspergillus brasiliensis* (AB), sélectionnées individuellement ou utilisées en mélange.

Le suivi de la qualité microbiologique des échantillons est réalisé par un comptage des microorganismes à différents temps : un jour après le traitement (J1), 7 jours après le traitement (J7), 14 jours après le traitement (J14), 28 jours après le traitement (J28).

A chaque temps, l'aptitude de préserver la composition contre une contamination microbiologique est jugée par la réduction des nombres des microorganismes, exprimé en log (ou, à 28 jours, par l'absence d'augmentation). Les critères A et B de la pharmacopée européenne, 8^{ème} édition (2016), paragraphe §5.1.3 pour les produits topiques applicables par voie cutanée sont les suivants :

**[Table 1]**

| Réduction (log) | | 2 jours | 7 jours | 14 jours | 28 jours |
|---|---|---|---|---|---|
| Bactéries | Critère A | *2 log* | *3 log* | | *Pas d'augmentation* |
| | Critère B | - | - | *3 log* | |
| Levures et moisissures | Critère A | - | - | *2 log* | |
| | Critère B | - | - | *1 log* | |

Vu le nombre des tests, uniquement la conformité ou non-conformité des compositions testées est indiqué sans détail des valeurs de réduction pour les différentes souches.

**[Table 2]**

| Conservateur testé | Concentrations testées | Résultats tests microbiologiques (conformité aux critères) | Commentaire / autres aspects |
|---|---|---|---|
| Formule sans conservateur | (control) | critères ni A ni B | |
| Hexetidine | 0.1% | ni A ni B | Formule colore (brunissement à la chaleur : 15 jours 50°c et 2 mois 40°C) |
| | 0.05% | ni A ni B | |
| Chloroxylenol | 0.5% | émulsion impossible à fabriquer | |
| | 0.1% | critères A | Forte odeur de crésol même à 0.01% |
| | 0.01% | ni A ni B | |
| | 0.001% | ni A ni B | |
| Chlorobutanol hemihydraté | 0.5% 0.1% | critères B ni A ni B | |
| Phenoxyéthanol | 1% | critères A | irritant |
| | 0.5% | ni A ni B | |
| Alcool benzylique | 1% | critères A | allergène |
| | 0.5% | ni A ni B | |
| Acide borique | nt | | irritant |
| Borate de sodium | nt | | irritant |
| Chlorure de benzalkonium | 0.25% | ni A ni B | allergisant |
| Triacetine | 1% | ni A ni B | |
| Alcool phenylethylique | 0.5% | ni A ni B | |
| | 0.25% | ni A ni B | |
| Pentyleneglycol | 5% | critères B | (attention lait fluide) |
| 1,2 hexanediol | 5% | critères A | (attention lait fluide) |
| | 1% | ni A ni B | |
| Sorbate de potassium | 0.2% | ni A ni B | odeur légère de sorbate |
| Propionate de sodium | 0.3% | ni A ni B | odeur piquante |
| 2-pyrrolidone | 5% | ni A ni B | Attention : jaunissement + odeur dès 3m40°C |
| | 7.5% | ni A ni B | |
| Octyldodecanol | 5% | ni A ni B | |
| Bleu de méthylène | 10ppm | ni A ni B | colorant est instable à la lumière |
| Thesit (macrogol 9 ether laurique) | 3% | ni A ni B | (attention très fluide) |
| Digluconate de cuivre | 5% | nt | Crème instable (déphasage à fabrication) |
| Undécylenate de zinc | 0.5% | critères B | Crème épaisse, aspect trié |
| Thymol | 0.5% | critères A | Forte odeur camphrée |
| Acide anisique | 0.5% | nt | Pb de dispersion et de recristallisation |
| Bisabolol | 1% | ni A ni B | |
| Capryloyl glycine | 2.5% | nt | Crème granuleuse |
| Undecylenol glycine | 2.5% | nt | Crème granuleuse |
| Oxyde de zinc (plusieurs préparations) | 1% | ni A ni B | Crème granuleuse, consistante |
| Steareth-2 | 1% | ni A ni B | |
| | 0.5% | ni A ni B | |
| Digluconate de CHX | 0.1% | ni A ni B | *CHX incriminé* |
| Bitrex | 10ppm (0.001%) | ni A ni B | |
| Sodium hydroxyméthylglycinate | 1% | critères A | libérateur de formol ! |
| Huile d'amandes douce raffinée | 1% | ni A ni B | |
| Parfum romacil | 0.5% | ni A ni B | |
| Chlorure de cetylpyridinium | 0.1% | ni A ni B | |
| Sodium acétate trihydrate | 0.5% | ni A ni B | |
| Hydroxyphenylpropamidobenzoic acid | 1% | nt | Déphasage |
| | | | |
| Polyquaternium 10 | 1% | ni A ni B | |
| Tryicapryline (Miglyol 808) | 5% | ni A ni B | |
| Propyl gallate | 0.1% | ni A ni B | |
| Acide pentétique | 0.1% | ni A ni B | léger granuleux d'aspect |
| | | | |

| | | | |
|---|---|---|---|
| conservateurs testes seuls | | | |

**[Table 3]**

| Conservateurs testé | Concentrations testées | Résultats tests microbiologiques (conformité aux critères) | Commentaire / autres aspects |
|---|---|---|---|
| Dioxyde de zinc A / propionate de sodium | 1% / 0.3% | ni A ni B | |
| Ethylhexylglycerine (EHG) / caprylyl glycol (=1,2-octanediol) | 0.5% / 0.3% | critères B | crème blanche liquide ou fluide |
| Ethylhexylglycerine (EHG) / pentylèneglycol | 0.25% / 0.4% | critères A | |
| | 0.2% / 0.4% | critères A | |
| | 0.15% / 0.4% | critères B | |

| | | | |
|---|---|---|---|
| conservateurs testés en combinaison. | | | |

Résultats : la plupart des conservateurs ne sont pas adaptés dans le corps de formule sous les conditions ciblées, pour diverses
raisons : critères microbiologiques, odeur, critères organoleptiques & physicochimiques, libération de formol, déphasage.

Les diols testés (pentyleneglycol, ethylhexylglycerine, hexanediol, caprylyl glycol) s'avèrent les plus compatibles.

### Exemple 2 : Tests des propriétés physicochimiques

### 2.1) Test à fabrication (TO)

Après les tests des différents systèmes conservateurs, il s'avère que très peu de produits sont adaptés dans les conditions particulières d'une composition comprenant une association de glycérol, vaseline et paraffine liquide. De plus, certains conservateurs efficaces entrainent une chute plus ou moins sévère de la consistance et/ou de la viscosité de l'émulsion. Une fluidification de la formule est notamment notée pour Pentyleneglycol, Ethylhexylglycerine, Hexanediol.

Pour étudier la possibilité de compenser les effets de fluidification, les différents conservateurs sont testés en présence et en absence d'un produit gélifiant, dans un premier temps un gélifiant de type polyacrylamide.

Pour étudier les propriétés galéniques, différents paramètres des compositions ont été suivies. Les caractères physico-chimiques des compositions ont été mesurés par les méthodes décrites ci-dessous. La taille des globules étant toujours inférieure à 25 µm, ce critère n'est pas représenté dans le tableau de résultats.

### Méthodes de caractérisation des compositions :

### Méthode de mesure de la consistance

La mesure de consistance se fait à température ambiante (environ 20°C) dans un pot en verre de 250 mL rempli à 200 g. La mesure se fait à l'aide d'un analyseur de texture type T AXT + (Swantec). La consistance du produit est représentée par la force maximale de résistance de la sonde dans le produit, qui est directement proportionnelle à la masse mesurée. La valeur de consistance est exprimée en g.

### Géométries de mesure :

Pot verre 250g rempli à 200g
Cylindre en acier P25
Paramètres de mesure :

| | |
|---|---|
| Pré-vitesse : | 4mm/s |
| Vitesse : | 8mm/s |
| Post vitesse : | 8mm/s |
| Déplacement : | 20mm |
| Seuil de déclenchement : | 5g |

Méthodologie de préparation de l'échantillon
Remplir le contenant (pot verre) sans introduire d'air dans le produit
Taper le pot de façon à tasser la crème et obtenir une surface la plus lisse possible.
Maintenir le pot fermé 24 avant de réaliser la mesure.

### Caractérisation microscopique

L'analyse est réalisée sur un microscope optique de type Olympus BX40, muni d'un objectif x40 (grossissement x 400)

### Méthodologie

Prélever la crème à l'aide d'une spatule, réaliser une prise d'essai répartie entre lame et lamelle et enregistrer un cliché photographique.

Sur le cliché calculer le nombre de globules total selon la méthode suivante :
- détermination du nombre de globules sur la ligne horizontale
- détermination du nombre de globules sur la ligne verticale.

Le nombre total de globules sur le cliché correspond à la multiplication des deux valeurs.

### Mesure de la viscosité

La mesure de viscosité se fait à température ambiante (environ 20°C) à l'aide d'un viscosimètre type Rheomat RM200. Elle est exprimée mPa·s.

### Méthode :

| | |
|---|---|
| Mobile : | Mobile 3 |
| Précisaillement (s⁻¹) : | / |
| Diamètre mobile : | Godet 1 |
| Cisaillement (s⁻¹) : | 7.6 s⁻¹ pendant 1 min |
| Température : | TA sans régulation |
| Type de mesure : | Pas à pas |
| Lecture : | à 60 secondes |

Formulation des compositions testés, en poids par rapport au poids de la composition :
environ 15 % de glycérol,
environ 8% de vaseline,
environ 2% de paraffine liquide,
environ 0,2 à 2% de trolamine,
environ 1 à 5% d'acide stéarique,
environ 2 à 10% de monostéarate de glycérol,
environ 0,5 à 2,5% de myristate d'isopropyle,
environ 0,2 à 2% de diméthicone,
environ 2 à 10% de polyéthylène glycol 600,
composants listés dans le tableau,
jusqu'à 100% en eau.

Les résultats à T0 des caractérisations physiques des essais de screening sont les suivants :

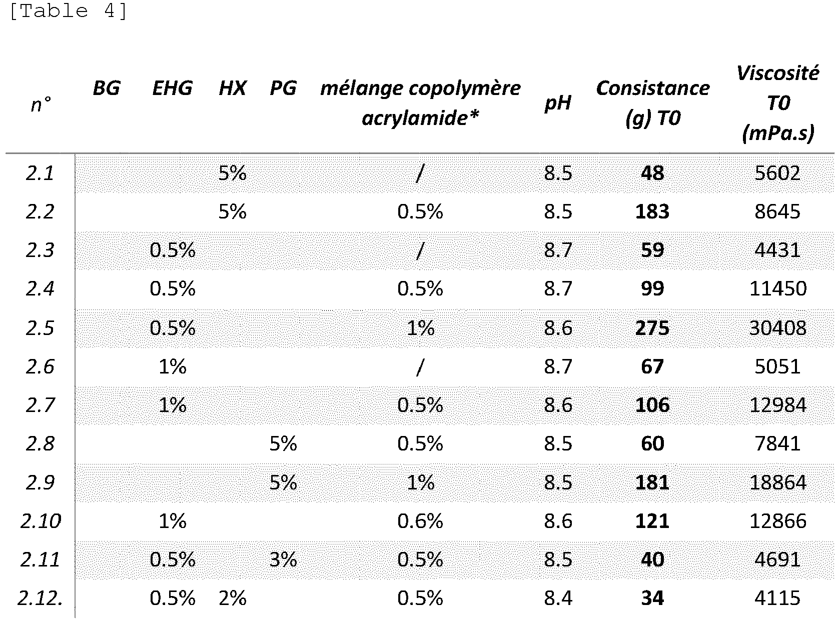

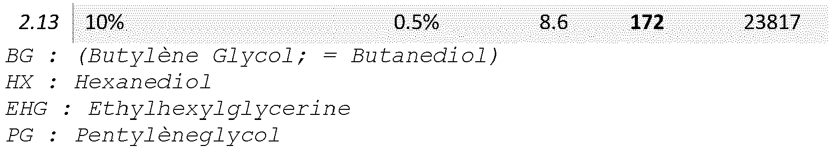

Pour ces exemples comparatifs, il s'avère que l'agent gélifiant de type polyacrylamide (ajouté à différentes concentrations) augmente la consistance et la viscosité des émulsions améliorant ainsi la texture en présence des conservateurs qui fluidifient la formule. Pour une quantité de 1%, on peut observer une surcompensation en termes de consistance dans un test.

La suite de l'évaluation consiste en test de stabilité des émulsions dans le temps à l'aide de certaines combinaisons sélectionnées, comme détaillé plus bas.
(* mélange copolymère acrylamide/sodium acryloyldiméthyltaurate avec isohexadecane et polysorbate, Sepineo^{®})

### 2.2) Suite de caractérisation des compositions : Stabilité à 3 mois 40°C 25%HR

Méthodes de mesure Consistance / Viscosité comme décrites en haut, à température ambiante.

Formulation des compositions testées, en poids par rapport au poids de la composition :
environ 15 % de glycérol,
environ 8% de vaseline,
environ 2% de paraffine liquide,
environ 0,2 à 2% de trolamine,
environ 1 à 5% d'acide stéarique,
environ 2 à 10% de monostéarate de glycérol,
0% ou environ 0,5 à 2,5% de myristate d'isopropyle,
environ 0,2 à 2% de diméthicone,
environ 2 à 10% de polyéthylène glycol 600,
composants listés dans le tableau,
jusqu'à 100% en eau.

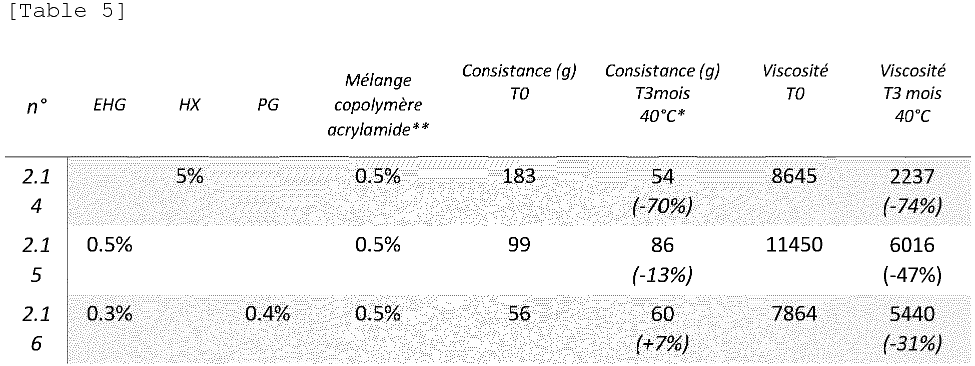

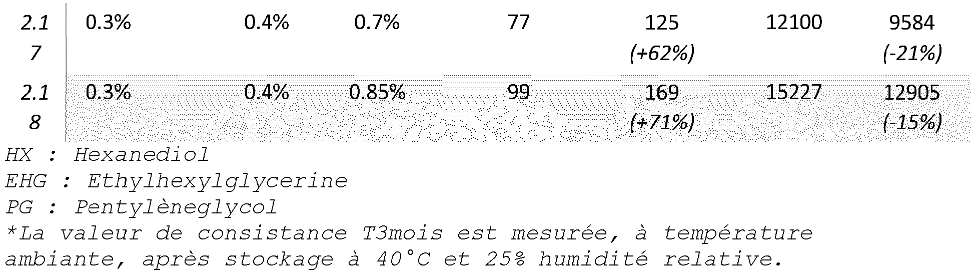

Pour ces exemples comparatifs, il s'avère que l'effet sur la texture des formules par le gélifiant de type copolymère polyacrylamide / le mélange acrylamide/sodium acryloyldiméthyltaurate avec isohexadecane et polysorbate n'est pas satisfaisant à long terme : il n'est pas durable ni prévisible dû à une maturation importante de l'émulsion, avec un grand écart des valeurs de consistance (> 45g) et/ou de la viscosité entre T0 et T3mois. De plus, la consistance et la viscosité sont faibles pour une formule.

Une compensation de ce phénomène a été recherchée par l'association d'un autre agent gélifiant, comme détaillé par la suite.
(** mélange copolymère acrylamide/sodium acryloyldiméthyltaurate avec isohexadecane et polysorbate, Sepineo^{®})

### Exemple 3. : Association agents gélifiants & conservateurs

### Exemple 3.1 : Test criblage agents gélifiants

Dans un premier temps, différentes combinaisons des agents gélifiants sont testées, en présence du conservateur hexanediol. Le but est de s'assurer que les propriétés physicochimiques ciblées peuvent être obtenues avec ces combinaisons.

Méthodes de caractérisation & corps de formules équivalent à l'exemple 2.1.

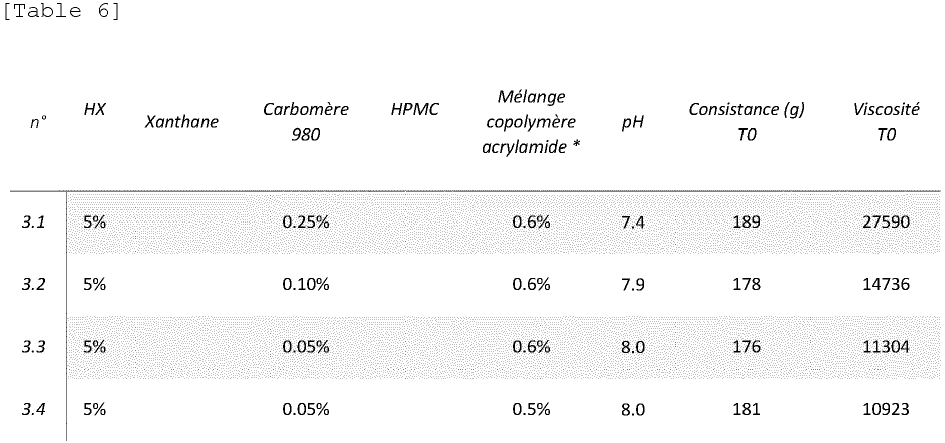

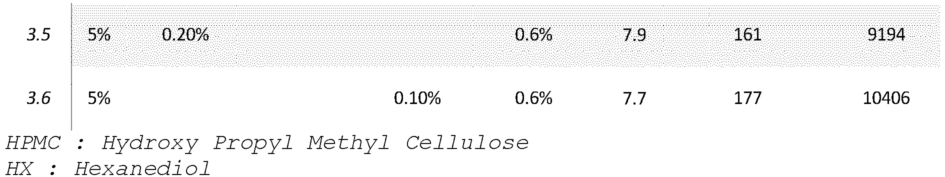

Résultat : A T0, les valeurs de consistance et viscosité sont acceptables pour les combinaisons testées. Dans la suite de l'évaluation, autres conservateurs et la stabilité à plus long terme sont inclus dans le test. Ceci est détaillé dans l'exemple 3.2.
(* mélange copolymère acrylamide/sodium acryloyldiméthyltaurate avec isohexadecane et polysorbate ; Sepineo^{®})

### Exemple 3.2 : Test association des gélifiants & conservateurs

Le but est de tester des diverses combinaisons de conservateurs et agents gélifiants pour tester leur compatibilité avec le corps de formule, et ceci pour la date de fabrication et pour la condition de stockage de 3 mois à température élevée (40°C)

### Méthodes de caractérisation équivalent à l'exemple 2.2

Formulation des compositions testés, en poids par rapport au poids de la composition :
environ 15 % de glycérol,
environ 8% de vaseline,
environ 2% de paraffine liquide,
environ 0,2 à 2% de trolamine,
environ 1 à 5% d'acide stéarique,
environ 2 à 10% de monostéarate de glycérol,
environ 0,5 à 2,5% de myristate d'isopropyle,
environ 0,2 à 2% de diméthicone,
environ 2 à 10% de polyéthylène glycol 600,
composants listés dans le tableau,
jusqu'à 100% en eau.

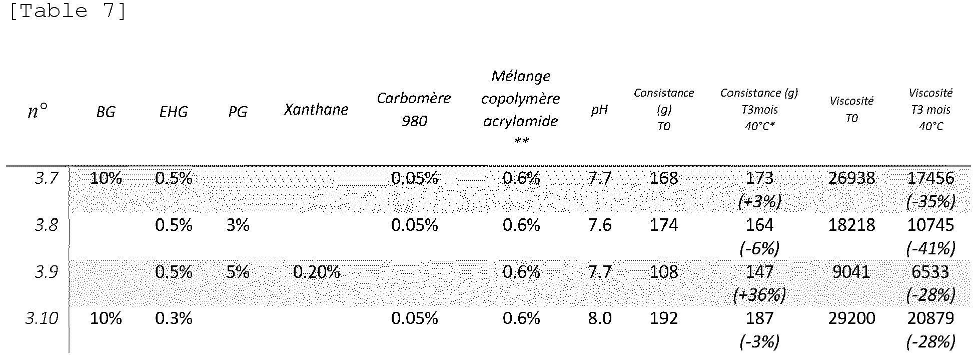

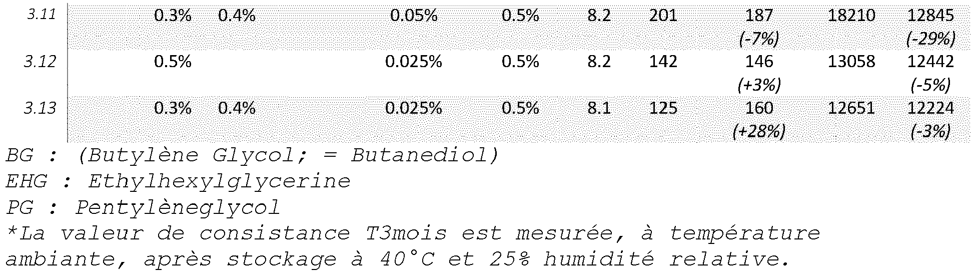

Suite à l'association des deux types des gélifiants (dont un gélifiant de type polyacrylamide, mélange copolymère acrylamide/sodium acryloyldiméthyltaurate avec isohexadecane et polysorbate ; Sepineo^{®}), les compositions sont bien adaptées en termes de consistance et viscosité pour différentes combinaisons des conservateurs, et ceci à T₀ ainsi que à T₃ₘₒᵢₛ (mesuré après stockage à 40°C et 25% humidité relative).
(** mélange copolymère acrylamide/sodium acryloyldiméthyltaurate avec isohexadecane et polysorbate ; Sepineo^{®})

## Revendications

1. Composition sous forme d'une émulsion huile dans eau ou eau dans huile, comprenant
- de l'eau à une concentration entre 30 et 80% en poids par rapport au poids total de la composition
- du glycérol à une concentration entre 10 et 20%, préférentiellement entre 13 et 17%, encore préférentiellement d'environ 15% en poids par rapport au poids total de la composition,
- de la vaseline à une concentration 3 et 20%, préférentiellement entre 5 et 10%, encore préférentiellement d'environ 8% en poids par rapport au poids total de la composition
- de la paraffine liquide à une concentration entre 0,5 et 5%, encore préférentiellement entre 1 et 3%, préférentiellement d'environ 2% en poids par rapport au poids total de la composition,
- au moins un conservateur, différent des parabènes, choisi dans le groupe consistant en l'hexanediol, l'éthylhexylglycérine, le pentylèneglycol, le butylèneglycol, le 1,2 octanediol (caprylyl glycol) et leurs mélanges,
- au moins deux agents gélifiants dont un agent gélifiant de type polyacrylamide et un second agent gélifiant choisi dans le groupe consistant en : gomme de xanthane, carbomère 980, carbomère 974, hydroxyéthylcelllulose, hydroxypropylméthylcellulose et leurs mélanges.

2. Composition selon la revendication 1, **caractérisée en ce que** le pH est compris entre pH 7,1 et pH 8,9, préférentiellement entre pH 7,4 et pH 8,7.

3. Composition selon l'une quelconque des revendications 1 à 2, **caractérisée en ce qu'**elle présente une consistance à fabrication comprise entre 70 et 230 g, d'une manière préférée entre 80 et 220 g à température ambiante.

4. Composition selon l'une quelconque des revendications 1 à 3, **caractérisée en ce qu'**elle présente une viscosité entre 7000 mPa·s et 40000 mPa·s, d'une manière préférée entre 9000 mPa·s et 30000 mPa·s, à température ambiante.

5. Composition selon l'une quelconque des revendications 1 à 4, **caractérisée en ce qu'**elle est stable dans le temps.

6. Composition selon l'une quelconque des revendications 1 à 5, **caractérisée en ce qu'**elle présente, après stockage pendant 3 mois à 40°C et 25% d'humidité relative,
- une valeur de consistance comprise entre comprise entre 40 et 210 g à température ambiante, de préférence entre 50 et 200 g,
- ainsi qu'une viscosité entre 6000 mPa·s et 30000 mPa·s à température ambiante.

7. Composition selon l'une quelconque des revendications 1 à 6, **caractérisée en ce que** ledit agent gélifiant de type polyacrylamide est un mélange copolymère acrylamide/sodium acryloyldiméthyltaurate avec isohexadecane et polysorbate.

8. Composition selon l'une quelconque des revendications 1 à 7, **caractérisée en ce qu'**elle contient un ou plusieurs agents d'étalement, de préférence choisi ou choisis dans le groupe consistant en diméthicone, polydiméthylcyclosiloxane, myristate d'isopropyle, palmitate d'isopropyle.

9. Composition selon l'une quelconque des revendications 1 à 8, pour son utilisation dans la prévention et/ou le traitement des xéroses iatrogènes et autres effets secondaires de traitements nécessitant l'application d'un émollient.

10. Composition selon l'une quelconque des revendications 1 à 8, pour son utilisation dans la prévention et/ou le traitement des xéroses apparues comme effets secondaires ou symptômes cutanés de pathologies telles que l'insuffisance rénale ou diabétique.

11. Composition selon l'une quelconque des revendications 1 à 8 pour son utilisation dans la prévention et/ou le traitement des états de sécheresse cutanée associés à certaines dermatoses telles que dermatite atopique, états ichtyosiques, psoriasis.

12. Composition selon l'une quelconque des revendications 1 à 8 pour son utilisation pour diminuer la fréquence et/ou réduire l'intensité des poussées eczémateuses observées chez les patients atteints de dermatite atopique.

13. Composition selon l'une quelconque des revendications 1 à 8 pour son utilisation dans la prévention et/ou le traitement des brûlures superficielles.

## Patentansprüche

1. Zusammensetzung in Form einer Öl-in-Wasser- oder Wasser-in-Öl-Emulsion, umfassend
- Wasser in einer Konzentration zwischen 30 und 80 Gew.-% bezogen auf das Gesamtgewicht der Zusammensetzung,
- Glycerin in einer Konzentration zwischen 10 und 20 Gew.-%, vorzugsweise zwischen 13 und 17 Gew.-%, noch vorzugsweise von etwa 15 Gew.-% bezogen auf das Gesamtgewicht der Zusammensetzung,
- Vaseline in einer Konzentration zwischen 3 und 20 Gew.-%, vorzugsweise zwischen 5 und 10 Gew.-%, noch vorzugsweise von etwa 8 Gew.-% bezogen auf das Gesamtgewicht der Zusammensetzung,
- flüssiges Paraffin in einer Konzentration zwischen 0,5 und 5 Gew.-%, noch vorzugsweise zwischen 1 und 3 Gew.-%, vorzugsweise von etwa 2 Gew.-% bezogen auf das Gesamtgewicht der Zusammensetzung,
- wenigstens ein Konservierungsmittel, das sich von Parabenen unterscheidet, das aus der Gruppe ausgewählt ist, die aus Hexandiol, Ethylhexylglycerin, Pentylenglycol, Butylenglycol, 1,2-Octandiol (Caprylylglykol) und deren Gemischen besteht,
- wenigstens zwei Geliermittel, darunter ein Geliermittel vom Typ Polyacrylamid und ein zweites Geliermittel, das aus der Gruppe ausgewählt ist, die besteht aus: Xanthangummi, Carbomer 980, Carbomer 974, Hydroxyethylcellulose, Hydroxypropylmethylcellulose und deren Gemischen.

2. Zusammensetzung nach Anspruch 1, **dadurch gekennzeichnet, dass** der pH-Wert zwischen pH 7,1 und pH 8,9, vorzugsweise zwischen pH 7,4 und pH 8,7 liegt.

3. Zusammensetzung nach einem der Ansprüche 1 bis 2, **dadurch gekennzeichnet, dass** sie eine Konsistenz bei Herstellung aufweist, die zwischen 70 und 230 g, bevorzugt zwischen 80 und 220 g bei Raumtemperatur liegt.

4. Zusammensetzung nach einem der Ansprüche 1 bis 3, **dadurch gekennzeichnet, dass** sie eine Viskosität aufweist, die zwischen 7000 mPa·s und 40000 mPa·s, bevorzugt zwischen 9000 mPa-s und 30000 mPa-s, bei Raumtemperatur liegt.

5. Zusammensetzung nach einem der Ansprüche 1 bis 4, **dadurch gekennzeichnet, dass** sie zeitstabil ist.

6. Zusammensetzung nach einem der Ansprüche 1 bis 5, **dadurch gekennzeichnet, dass** sie nach einer Lagerung von 3 Monaten bei 40 °C und 25 % relativer Luftfeuchtigkeit aufweist
- einen Konsistenzwert zwischen 40 und 210 g bei Raumtemperatur, vorzugsweise zwischen 50 und 200 g,
- sowie eine Viskosität zwischen 6000 mPa-s und 30000 mPa-s bei Raumtemperatur.

7. Zusammensetzung nach einem der Ansprüche 1 bis 6, **dadurch gekennzeichnet, dass** das Geliermittel vom Typ Polyacrylamid ein Copolymergemisch Acrylamid/Natriumacryloyldimethyltaurat mit Isohexadecan und Polysorbat ist.

8. Zusammensetzung nach einem der Ansprüche 1 bis 7, **dadurch gekennzeichnet, dass** sie ein oder mehrere Spreitmittel enthält, das oder die vorzugsweise aus der Gruppe ausgewählt ist/sind, die aus Dimethicon, Polydimethylcyclosiloxan, Isopropylmyristat, Isopropylpalmitat besteht.

9. Zusammensetzung nach einem der Ansprüche 1 bis 8 für ihre Verwendung bei der Prävention und/oder Behandlung von iatrogenen Xerosen und anderen Nebenwirkungen von Behandlungen, die die Anwendung eines Weichmachers erfordern.

10. Zusammensetzung nach einem der Ansprüche 1 bis 8 für ihre Verwendung bei der Prävention und/oder Behandlung von Xerosen, die als Nebenwirkungen oder Hautsymptome von Erkrankungen wie Niereninsuffizienz oder Diabetes auftreten.

11. Zusammensetzung nach einem der Ansprüche 1 bis 8 für ihre Verwendung bei der Prävention und/oder Behandlung von Hauttrockenheitszuständen im Zusammenhang mit bestimmten Dermatosen wie atopischer Dermatitis, ichtyosischen Zuständen, Psoriasis.

12. Zusammensetzung nach einem der Ansprüche 1 bis 8 für ihre Verwendung zur Verringerung der Häufigkeit und/oder Verringerung der Intensität von ekzematösen Schüben, die bei Patienten mit atopischer Dermatitis beobachtet werden.

13. Zusammensetzung nach einem der Ansprüche 1 bis 8 für ihre Verwendung bei der Prävention und/oder Behandlung von oberflächlichen Verbrennungen.

## Claims

1. A composition in the form of an oil-in-water or water-in-oil emulsion, comprising
- water at a concentration between 30 and 80% by weight relative to the total weight of the composition
- glycerol at a concentration between 10 and 20%, preferably between 13 and 17%, still preferably about 15% by weight relative to the total weight of the composition,
- vaseline at a concentration between 3 and 20%, preferably between 5 and 10%, still preferably about 8% by weight relative to the total weight of the composition
- liquid paraffin at a concentration between 0.5 and 5%, still preferably between 1 and 3%, preferably about 2% by weight relative to the total weight of the composition,
- at least one preservative, other than parabens, selected from the group consisting of hexanediol, ethylhexylglycerin, pentyleneglycol, butyleneglycol, 1,2 octanediol (caprylyl glycol) and mixtures thereof,
- at least two gelling agents including a gelling agent of the polyacrylamide type and a second gelling agent selected from the group consisting of: xanthan gum, carbomer 980, carbomer 974, hydroxyethylcellulose, hydroxypropylmethylcellulose and mixtures thereof.

2. The composition according to claim 1, **characterised in that** the pH is between pH 7.1 and pH 8.9, preferably between pH 7.4 and pH 8.7.

3. The composition according to any one of claims 1 to 2, **characterised in that** it has a consistency at manufacture of between 70 and 230 g, preferably between 80 and 220 g at room temperature.

4. The composition according to any one of claims 1 to 3, **characterised in that** it has a viscosity between 7,000 mPa·s and 40,000 mPa·s, preferably between 9,000 mPa·s and 30,000 mPa·s, at room temperature.

5. The composition according to any one of claims 1 to 4, **characterised in that** it is stable over time.

6. The composition according to any one of claims 1 to 5, **characterised in that** it has, after storage for 3 months at 40°C and 25% relative humidity,
- a consistency value between 40 and 210 g at room temperature, preferably between 50 and 200 g,
- as well as a viscosity between 6,000 mPa·s and 30,000 mPa-s at room temperature.

7. The composition according to any one of claims 1 to 6, **characterised in that** said gelling agent of the polyacrylamide type is an acrylamide/sodium acryloyldimethyltaurate copolymer mixture with isohexadecane and polysorbate.

8. The composition according to any one of claims 1 to 7, **characterised in that** it contains one or more spreading agents, preferably selected from the group consisting of dimethicone, polydimethylcyclosiloxane, isopropyl myristate and isopropyl palmitate.

9. The composition according to any one of claims 1 to 8, for use in the prevention and/or treatment of iatrogenic xerosis and other side effects of treatments requiring the application of an emollient.

10. The composition according to any one of claims 1 to 8, for use in the prevention and/or treatment of xerosis appearing as side effects or skin symptoms of pathologies such as kidney or diabetic failure.

11. The composition according to any one of claims 1 to 8, for use in the prevention and/or treatment of dry skin conditions associated with some dermatoses such as atopic dermatitis, ichthyosis states and psoriasis.

12. The composition according to any one of claims 1 to 8, for use in decreasing frequency and/or reducing intensity of eczema flares observed in patients with atopic dermatitis.

13. The composition according to any one of claims 1 to 8, for use in the prevention and/or treatment of superficial burns.
